(19) Europäisches Patentamt

European Patent Office

Office européen des brevets

(11) **EP 0 845 978 B1**

(12) **EUROPÄISCHE PATENTSCHRIFT**

(45) Veröffentlichungstag und Bekanntmachung des Hinweises auf die Patenterteilung:
**27.02.2002 Patentblatt 2002/09**

(21) Anmeldenummer: **96929227.5**

(22) Anmeldetag: **09.08.1996**

(51) Int Cl.$^7$: **A61K 7/32**

(86) Internationale Anmeldenummer:
**PCT/EP96/03520**

(87) Internationale Veröffentlichungsnummer:
**WO 97/06776 (27.02.1997 Gazette 1997/10)**

(54) **TRANSLUCENTE ANTITRANSPIRANTIEN/DEODORANTIEN**

TRANSLUCENT ANTIPERSPIRANTS/DEODORANTS

ANTI-TRANSPIRANTS/DEODORANTS TRANSLUCIDES

(84) Benannte Vertragsstaaten:
**AT BE CH DE DK ES FI FR GB GR IE IT LI NL PT SE**

(30) Priorität: **17.08.1995 DE 19530220**

(43) Veröffentlichungstag der Anmeldung:
**10.06.1998 Patentblatt 1998/24**

(73) Patentinhaber: **Henkel Kommanditgesellschaft auf Aktien**
**40589 Düsseldorf-Holthausen (DE)**

(72) Erfinder:
• **FÖRSTER, Thomas**
**40699 Erkrath (DE)**
• **CLAAS, Marcus**
**40723 Hilden (DE)**
• **BANOWSKI, Bernhard**
**40597 Düsseldorf (DE)**

(56) Entgegenhaltungen:
**WO-A-93/07249**      **DE-A- 4 337 041**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

**Gebiet der Erfindung**

Die Erfindung betrifft translucente Antitranspirantien/Deodorantien auf Basis feinteiliger, sprühfähiger Mikroemulsionen. Weiterhin betrifft die Erfindung Mikroemulsionskonzentrate sowie ein Verfahren zur Herstellung translucenter Antitranspirantien/Deodorantien aus solchen Konzentraten. Die erfindungsgemäßen, stabilen Mikroemulsionen besitzen dabei im wesentlichen einen Tröpfchendurchmesser von weniger als 100 nm.

**Stand der Technik**

Die Verwendung von Öl-in-Wasser-Emulsionen als Träger für kosmetische und dermatologische Wirkstoffe ist seit langem bekannt. Eine Schwierigkeit besteht darin, daß durch die Natur der Wirkstoffe die Stabilität des Trägersystems oft nachteilig beeinflußt wird. Aus der deutschen Patentanmeldung **P 43 37 041.1** war bereits bekannt, daß sich sehr feinteilige Emulsionen, die nach dem Phaseninversions-Verfahren hergestellt sind, als Träger für organische Deodorantien, Parfümöle und Lichtschutzfaktoren besonders gut eignen. Aus dieser Druckschrift konnte jedoch nicht der Schluß gezogen werden, daß solche Emulsionen auch als Träger für wasserlösliche, anorganische, adstringierende Antitranspirant-Wirkstoffe geeignet sind.

Es bestand die Aufgabe, Antitranspirantien in ihrer Haut- und Schleimhautverträglichkeit dadurch zu verbessern, daß man als Träger für die adstringierenden Wirkstoffe eine Emulsion verwendet, deren Ölkomponenten auf die Haut einen weichmachenden und entzündungswidrigen Effekt ausüben. Die besonders gute Verträglichkeit sollte dadurch erreicht werden, daß eine möglichst feinverteilte Emulsion mit möglichst geringer Tröpfchengröße als Träger des Antitranspirant-Wirkstoffs zur Verfügung gestellt wird. Das Problem bei solchen feinverteilten Emulsionen bestand vor allem darin, daß die üblichen Öl-in-Wasser-Emulsionen in Gegenwart der anorganischen Wirkstoffe nicht über eine ausreichende Stabilität verfügen, und die Viskosität der Mikroemulsionen mit abnehmender Tröpfchengröße in der Regel so stark anstieg, daß eine Verarbeitung in Form einer sprühfertigen Trägersubstanz nicht möglich war.

Es wurde nun überraschend festgestellt, daß das Problem dadurch gelöst werden kann, daß man als Träger eine sehr feinteilige Emulsion verwendet, wie sie sich beispielsweise durch Einsatz großer Mengen geeigneter nichtionischer Tenside gegebenenfalls in Verbindung mit Coemulgatoren erreichen läßt. Die Erfindung stellt sowohl translucente, feinteilige als auch niedrigviskose und damit sprühfähige Antitranspirantien zur Verfügung.

Als Antitranspirantien wirksame Substanzen entfalten in der Regel auch eine Desodorierende Wirkung, weshalb unter Antitranspirantien im Sinne der vorliegenden Erfindung auch solche Zusammensetzungen zu verstehen sind, bei denen im wesentlichen die desodorierende Wirkung im Vordergrund steht. Dies kann Beispielsweise bei Zusammensetzungen der Fall sein, die den Antitranspirant-Wirkstoff in so geringen Konzentrationen enthalten, daß die desodorierende Komponente des Wirkungsspektrums gegenüber der antitranspirativ wirksamen Komponente überwiegt. Im folgenden Text steht deshalb die Bezeichnung Antitranspirantien synonym für Antitranspirantien/Deodorantien oder Antitranspirantien und Deodorantien.

Gegenstand der Erfindung sind daher translucente Antitranspirantien in Form einer sprühfähigen, feinteiligen Öl-in-Wasser-Emulsion, enthaltend

a) 0,5 bis 30 Gew.-% einer nicht mit Wasser mischbaren Ölphase,
b) 40 bis 90 Gew.-% Wasser und
c) bezogen auf einen Gewichtsteil Ölphase, 0,35 bis 30 Gew.-Teile nichtionischer Emulgatoren, insbesondere aus der Gruppe der Alkylpolyglykoside,
d) gegebenenfalls 0,1 bis 7,5 Gew.-Teile eines lipophilen Coemulgators sowie weitere übliche Zusatzstoffe,

dadurch gekennzeichnet, daß weiterhin 0,1 bis 25 Gew.-% eines anorganischen, adstringierenden Antitranspirant-Wirkstoffes enthalten sind und die Tröpfchengröße der emulgierten Phase im wesentlichen zwischen 10 und 100 nm liegt.

Solche erfindungsgemäßen Antitranspirant-Formulierungen zeichnen sich durch eine besonders gute Hautverträglichkeit des Antitranspirant-Wirkstoffes aus, die auch bei regelmäßiger Anwendung erhalten bleibt.

Als wasserlösliche, anorganische, adstringierende Antitranspirant-Wirkstoffe eignen sich vor allem Salze des Aluminiums, Zirkoniums oder des Zinks.

Solche geeigneten antihydrotisch wirksamen Wirkstoffe sind zum Beispiel Aluminiumchlorid, Aluminiumchlorhydrat, Aluminiumdichlorhydrat, Aluminiumsesquichlorhydrat und deren Komplexverbindungen mit 1,2-Propylenglycol, Aluminiumhydroxyallantoinat, Aluminiumchlorid-tartrat, Aluminium-Zirkonium-Trichlorohydrat, Aluminium-Zirkonium-tetrachlorohydrat, Aluminium-Zirkonium-pentachlorohydrat und deren Komplexverbindungen mit Aminosäuren z.B. mit Glycin.

Bevorzugt eignen sich jedoch Aluminium-hydroxychloride und deren Addukte an wasserlösliche Glycole. Als wasserlöslich werden dabei solche Salze verstanden, die bei 20 °C zumindest zu 1 Gew.-% in Wasser löslich sind.

Die wasserlöslichen, anorganischen, adstringierenden Antitranspirant-Wirkstoffe werden erfindungsgemäß in Mengen von 0,1 bis 25 Gew.-% eingesetzt. In bestimmten Fällen ist es jedoch vorteilhaft, die wasserlöslichen, anorganischen, adstringierenden Antitranspirant-Wirkstoffe in Mengen von 1 bis 15 Gew.-%, bevorzugt von 2 bis 10 Gew.-% und insbesondere bevorzugt von 4 bis 9 Gew.-% einzusetzen. Bestimmte Antitranspirans-Formulierungen können dagegen auch 0,1 bis 10 Gew.-% oder bevorzugt 1 bis 6 Gew.-% des Wirkstoffs enthalten, während Formulierungen, beispielsweise in Konzentratform, durchaus Wirkstoffmengen von 8 bis 25 Gew.-%, bevorzugt 10 bis 22 Gew.-% und insbesondere bevorzugt 10 bis 18 Gew.-% enthalten können.

Träger des Antitranspirant-Wirkstoffs sind sogenannte Mikroemulsionen. Mikroemulsionen sind optisch isotrope, thermodynamisch stabile Systeme, die eine wasserunlösliche Ölkomponente, Emulgatoren und Wasser enthalten. Das klare bzw. transparente Aussehen der Mikroemulsionen ist eine Folge der geringen Tröpfchengröße der dispergierten Emulsionströpfchen, die im wesentlichen unter 100 nm, im Mittel immer unter 50 nm liegt.

Mikroemulsionen sind in der Literatur häufig beschrieben, ihre gezielte Herstellung ist aber mit großen Schwierigkeiten verbunden, da die Existenzbereiche der Mikroemulsionen in dem aus Ölkomponente, Wasser und Emulgatoren gebildeten 3-Phasen-Diagramm meist sehr klein sind und die Lage dieser Existenzbereiche in hohem Maße von strukturellen Merkmalen aller Komponenten und aller weiteren Inhaltsstoffe solcher Systeme stark beeinflußt wird.

Die Methoden zur Herstellung von Mikroemulsionen sind im Prinzip bekannt. Man stellt Mischungen aus Wasser, Ölkomponente und Emulgatoren her und bestimmt die optisch isotropen, thermodynamisch stabilen Existenzbereiche in dem aus diesen Komponenten gebildeten 3-Phasen-Diagramm. Als kosmetische Ölkomponenten, im nachfolgenden Ölphase genannt, eignen sich alle wasserunlöslichen, hautverträglichen Öle und Fettstoffe und deren Mischungen mit festen Paraffinen und Wachsen. Als hautverträgliche Ölkomponenten eignen sich bevorzugt bei 20 °C noch flüssige Kohlenwasserstoffe, z.B. Paraffinöle und synthetische Kohlenwasserstoffe wie z.B. 1,3-Di-(2-ethyl-hexyl)-cyclohexan. Weiterhin besonders geeignete Ölkomponenten sind die Di-n-alkylether wie z.B. Di-n-octylether, Di-(2-ethyl-hexyl)-ether, Lauryl-Methylether oder Octylbutylether.

Eine besonders vielseitige Gruppe von kosmetischen Ölkomponenten ist die der Fettsäure- und Fettalkoholester, z.B. Isopropylmyristat, n-Butylstearat, 2-Ethylhexylcaprylat, Cetyloleat, Glycerintricaprylat, Kokosfettalkohol-($C_{12}$-$C_{18}$)-Caprylat-/Caprinat und andere.

Geeignet sind auch natürlich vorkommende Esteröle wie z.B. Jojobaöl oder flüssige, pflanzliche Triglyceridöle, wie z.B. Olivenöl, Sonnenblumenöl, Sojaöl, Rapsöl, Mandelöl, die flüssigen Anteile des Kokosöls oder des Rindertalgs sowie synthetische Triglyceridöle. Ebenso geeignet sind die Dicarbonsäureester wie beispielsweise Di-n-butyl-adipat, Di-n-butyl-sebazat, Di-(2-ethylhexyl)-adipat, Di-(2-hexyldecyl)succinat und Di-isotridecyl-azelaat. Geeignete Diolester sind z.B. Ethylenglycoldioleat, Ethylenglycol-di-isotridecanoat, Propylenglycol-di-(2-ethylhexanoat), Propylenglycol-diisostearat, Propylenglycol-di-pelargonat, Butandiol-diisostearat und Neopentylglycol-dicaprylat.

Bevorzugt werden als Bestandteil der Ölphase die Di-n-alkylether eingesetzt, wobei der Einsatz von Di-n-octylether besonders bevorzugt ist. Die Di-n-alkylether sind dabei in einem Anteil von mindestens 30 Gew.-% bevorzugt 40 Gew.-% und insbesondere bevorzugt wenigstens 50 Gew.-% enthalten. Besonders vorteilhafte Ausführungsformen der Erfindung enthalten Di-n-alkylether in der Ölphase in einem Anteil von wenigstens 60 %, wobei es vorteilhaft sein kann, den Anteil an Ether auf beispielsweise 80 oder 90 Gew.-% zu erhöhen. Gegebenenfalls kann der Anteil der Ether an der Ölphase sogar 100 Gew.-% betragen, wobei die Phasenbreite der Mikroemulsion hier besonders groß ist.

Die erfindungsgemäßen Antitranspirantien können die Ölphase vorteilhafterweise in einem Anteil von 2 bis 15 Gew.-%, bevorzugt 2 bis 10 Gew.-% am gesamten Antitranspirans in der Emulsion enthalten.

Der Wasseranteil in der Emulsion des fertigen Antitranspirans beträgt üblicherweise zwischen 40 und 90 Gew.-%. In der Regel ist es notwendig aufgrund der geringen Phasenbreiten der Emulsion den Wassergehalt zu variieren, wobei sich für die erfindungsgemäßen Antitranspirantien ein Wassergehalt von 60 bis 90 Gew.-%, insbesondere bevorzugt ein Wassergehalt zwischen 70 und 88 Gew.-% als vorteilhaft erwiesen hat.

Als Emulgatoren enthalten die Mikroemulsionen üblicherweise einen hydrophilen, nichtionischen Emulgator mit einem HLB-Wert von bevorzugt 8 bis 15. Unter HLB-Wert soll dabei ein Größe verstanden werden, die aus der Struktur des Emulgators errechnet werden kann gemäß

$$HLB = \frac{100\text{-}L}{5} \qquad\qquad I$$

worin L der Gewichtsanteil (in %) der liphophilen Gruppen, d.h. der Fettalkyl- oder Fettacylgruppen im Emulgator ist.

Bei den hydrophilen Emulgatoren handelt es sich beispielsweise um Anlagerungsprodukte von Ethylenoxid an Fettalkohole mit 16 bis 22 C-Atomen oder an Partialester von Polyolen mit 3 bis 6 C-Atomen und Fettsäuren mit 14 bis 22 C-Atomen. Geeignet sind aber auch Anlagerungsprodukte von Ethylenoxid an Fettsäuren, an Alkylglycoside, an Methylglycosid-Fettsäureester, an Fettsäurealkanolamide, an Fettsäure-N-alkylpolyhydroxyalkylamide und andere Fettstoffe mit ethoxylierbaren Substituenten.

Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab (Fettsäureglucamide).

Erfindungsgemäß besonders bevorzugt ist jedoch der Einsatz von Alkylpolyglycosiden der Formel

$$RO-(Z)_X,$$

in der R einen $C_8$- bis $C_{22}$-Alkyl- oder Alkenylrest, Z ein Monosaccharid, insbesondere Glucose, und x dessen Oligomerisationsgrad, eine Zahl von 1,1 bis 1,5, insbesondere von 1,2 bis 1,4, darstellt. Die Herstellung solcher Alkylpolyglycoside und ihre Verwendung als oberflächenaktive Stoffe sind beispielsweise aus der **DE-19 43 689** oder aus **DE-38 27 543** bekannt. Ihre Herstellung erfolgt durch Umsetzung von Glucose oder Oligosacchariden mit primären Alkoholen mit 8 bis 16 C-Atomen oder durch Umacetalisierung von Stärke mit z.B. niederen Alkoholen und erneute Umacetalisierung mit dem $C_8$-$C_{14}$-Fettalkohol.

Die nichtionischen Emulgatoren, insbesondere diejenigen aus der Gruppe der Alkypolyglycoside, werden in der Regel in einen Anteil von 0,35 bis 30 Gewichtsteilen pro Gewichtsteil der Ölphase, bevorzugt in einem Anteil von 0,5 bis 10 Gewichtsteilen und insbesondere bevorzugt in einem Anteil von 0,6 bis 4 Gewichtsteilen pro Gewichtsteil Ölphase eingesetzt. Erfindungsgemäß besonders vorteilhafte Ergebnisse lassen sich jedoch erzielen, wenn man die nichtionischen Emulgatoren in einem Anteil von 0,7 bis 1,4 Gewichtsteilen pro Gewichtsteil Ölphase zum Einsatz bringt. In der Regel ist es hierbei am günstigsten, einen oder mehrere nichtionische Emulgatoren aus der Gruppe der Alkylpolyglycoside mit einer Kettenlänge des lipophilen Rests zwischen 8 und 16 C-Atomen einzusetzen.

Gegebenenfalls kann es notwendig sein, zur Erzielung der gewünschten feinteiligen Emulsionen einen oder mehrere lipophile Coemulgatoren der Ölphase zuzumischen. Als lipophile Coemulgatoren können beispielsweise Fettsäure-Polyol-Partialester, Fettalkohole oder Fettalkohol-Polyol-Ether verwendet werden.

Unter Fettsäure-Polyol-Partialestern versteht man die Produkte der Veresterung von Fettsäuren mit 8 bis 22 C-Atomen, vorzugsweise 12 bis 22 C-Atomen mit polyfunktionellen Alkoholen der Funktionalität 3 bis 10, vorzugsweise 3 bis 6 und insbesondere bevorzugt 3 oder 4.

Besonders bevorzugt sind dabei solche Fettsäure-Polyol-Partialester, bei denen lediglich eine OH-Funktionalität mit einer geeigneten Fettsäure verestert ist. Die bei einer solchen Veresterung entstehenden Gemische sollten vorteilhafterweise 35 bis 96 % Monoester, 1 bis 50 % Diester und 0,1 bis 20 % Tri- oder höhere Ester enthalten.

Wählt man Glycerin als Polyol, so lassen sich die Partialester auf besonders einfache Weise durch Umesterung natürlicher Fette oder Öle mit einem Überschuß an Glycerin erhalten. Geeignete natürliche Fette und Öle stellen beispielsweise Rindertalg, Schweinschmalz, Palmöl, Sonnenblumenöl oder Sojaöl, bevorzugt solche natürlichen Fette oder Öle mit einem besonders hohen Anteil an Ölsäure dar. Als Polyole eignen sich z.B. Propylenglycol, Glycerin, Erythrittrimethylolpropan, Pentaerythrit, Sorbit, Diglycerin, Methylglycosid oder auch Aldosen wie z.B. Glucose oder Mannose.

Geeignete Fettsäure-Polyol-Partialester sind z.B. die technischen Glycerin- oder Sorbitan-Monoester von Myristinsäure, Palmitinsäure, Stearinsäure und Ölsäure oder von technischen Kokosfettsäure $C_{12}$-$C_{18}$-Schnitten. Bevorzugt werden Sorbitanmonooleat, Stearinsäuremonoglycerid sowie insbesondere bevorzugt Ölsäuremonoglycerid verwendet.

Ebenso geeignet ist der Einsatz von linearen und/oder verzweigten Fettalkoholen mit 8 bis 22 C-Atomen die gegebenenfalls eine oder mehrere Doppelbindungen in der Kohlenstoffkette enthalten können, sowie Partialether von polyfunktionellen Alkoholen wie sie bei der Beschreibung der Fettsäure-Polyol-Partialester erwähnt wurden mit Fettalkoholen mit 8 bis 22 C-Atomen.

Der lipophile Coemulgator wird gegebenenfalls in einer Menge von 0,01 bis 7,5 Gewichtsteilen pro Gewichtsteil Ölphase eingesetzt, wobei eine Menge von 0,1 bis 5 und insbesondere eine Menge von 0,2 bis 3 Gewichtsteilen pro Gewichtsteil Ölphase bevorzugt ist. In vielen Fällen kann es von Vorteil sein, den lipophilen Coemulgator in eine Menge von 0,2 bis 1,4 oder bevorzugt 0,2 bis 1,2 Gewichtsteilen pro Gewichtsteil Ölphase einzusetzen.

Das Verhältnis von nichtionischem Emulgator zu Coemulgator sollte in der Regel zwischen 1 : 1 und 20 : 1 liegen, wobei es bevorzugt ist Einsatzverhältnisse zwischen 2 : 1 und 15 : 1 bzw. 2 : 1 und 10 : 1 wählen. Besonders vorteilhafte Ergebnisse lassen sich erzielen, wenn das Vehältnis von nichtionischem Emulgator zu Coemulgator zwischen 2,5 : 1 und 5 : 1 liegt.

In vielen Fällen kann jedoch durch die coemulgierende Wirkung des gegebenenfalls eingesetzten Parfümöls auf die Zugabe von Coemulgatoren verzichtet werden.

Die erfindungsgemäßen, translucenten Antitranspirantien können neben den bislang angesprochenen Inhaltsstoffen noch weitere, übliche Zusatzstoffe enthalten. Hierbei handelt es sich beispielsweise um wasserlösliche Polyole mit 2 bis 8 C-Atomen und 2 bis 6 Hydroxylgruppen. Solche geeigneten Polyole sind z.B. Ethylenglycol, 1,2-Propylenglycol, Glycerin, Erythrit, Trimethylolpropan, Sorbit oder Methylglycosid. Anstelle dieser Polyole können auch Polyethylenglycole oder Anlagerungsprodukte von Ethylenoxid an solche Polyole verwendet werden.

Niedere, flüchtige Alkohole sollten nur in geringen Mengen, bevorzugt aber gar nicht in den erfindungsgemäßen Antitranspirantien enthalten sein. So sollte z.B. der Gehalt an Ethanol oder Isopropanol einen Wert von 2 Gew.-% nicht überschreiten.

Daneben können aber in den erfindungsgemäßen Antitranspirantien übliche öllösliche und/oder wasserlösliche Hilfsmittel in geringen Mengen enthalten sein. Solche öllöslichen Hilfsmittel können z.B. entzündungshemmende, hautschützende oder wohlriechende äterische Öle, synthetische, hautschützende Wirkstoffe, oder öllösliche Parfümöle sein. Zu dieser Gruppe zählen z.B. auch die öllöslichen Deowirkstoffe wie z.B. Triethylcitrat, Phenoxyethanol und/oder 2,4,4'-Trichloro-2'-hydroxydiphe-nylether, die der erfindungsgemäßen Emulsion, gegebenenfalls auch im Gemisch mit öllöslichen Konservierungsmitteln wie beispielsweise PHB-Estern, zugesetzt werden können.

Übliche wasserlösliche Zusätze sind z.B. Konservierungsmittel, öl- oder wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon, Poly(meth)acrylate oder hochmolekulare Polyethylenoxide.

Ein weiteres Gebiet der Erfindung betrifft die Herstellung translucenter Antitranspirantien. Die Herstellung erfolgt in der Regel über ein zweistufiges Kaltverfahren, wobei zunächst ein wasserverdünnbares Mikroemulsionskonzentrat hergestellt wird.

Die Erfindung betrifft daher auch translucente Mikroemulsionskonzentrate, enthaltend

a) 20 bis 40 Gew.-% Wasser sowie
b) 20 bis 40 Gew.-% eines nicht wasserlöslichen Ölkörpers und
c) bezogen auf einen Gewichtsteil der Ölphase, 0,35 bis 30 Gew.-Teile nichtionischer Emulgatoren, insbesondere aus der Gruppe der Alkylpolyglycoside sowie
d) gegebenenfalls 0,1 bis 10 Gew.-% Parfümöle,

dadurch gekennzeichnet, daß die Tröpfchengröße im wesentlichen zwischen 1 und 100 nm liegt.

Die einzelnen Bestandteile entsprechen hierbei denen, die schon im Rahmen der Beschreibung der erfindungsgemäßen Antitranspirantien erläutert wurden.

Ein weiterer Gegenstand der Erfindung ist ein Verfahren zur Herstellung translucenter Mikroemulsionskonzentrate, das dadurch gekennzeichnet ist, daß man die wasserlöslichen Komponenten in Wasser, die öllöslichen Komponenten (gegebenenfalls bei erhöhter Temperatur) in der Ölphase löst und die Phasen bei Temperaturen zwischen 10 und 90°C, insbesondere zwischen 20 und 70°C intensiv vermischt.

Kommen Coemulgatoren zum Einsatz, die zwischen 10 und 30°C flüssig sind, so kann eine Verarbeitung meist bei Raumtemperatur erfolgen, wobei unter Raumtemperatur im Sinne der Beschreibung eine Temperatur zwischen 20 und 25°C zu verstehen ist. Eine Temperaturerhöhung kann jedoch bei der Herstellung der Mikroemulsionen insbesondere dann erforderlich sein, wenn ein lipophiler, wachsartiger Coemulgator mit erhöhtem Schmelzpunkt eingesetzt wird. So muß beispielsweise beim Einsatz von Glycerinmonooleat als Coemulgator die Herstellung des erfindungsgemäßen Mikroemulsionskonzentrats durch Vermischen der wäßrigen- und der Ölphase bei einer Temperatur von 65°C erfolgen. Nach dem Abkühlen erhält man ein niedrigviskoses System, in welches gewünschtenfalls Zusatzstoffe wie beispielsweise Parfümöl eingerührt werden können. Hierbei bildet sich üblicherweise eine wasserklare niedrigviskose Mikroemulsion, die als Konzentrat in einem zweiten Verfahrensschritt zur Herstellung der erfindungsgemäßen translucenten Antitranspirantien zur Verfügung steht.

Gegenstand der Erfindung ist daher auch ein Verfahren zur Herstellung translucenter Antitranspirantien, dadurch gekennzeichnet, daß ein translucentes Mikroemulsionskonzentrat mit einer wäßrigen Lösung von Antitranspirant-Wirkstoffen intensiv vermischt wird.

Die folgenden Beispiele sollen den Gegenstand der Erfindung näher erläutern, ohne ihn jedoch auf den Inhalt der Beispiele einzuschränken.

**Beispiele**

Liste der verwendeten Inhaltsstoffe:

Plantaren® 1200 : $C_{12}$- $C_{16}$- Alkyl-oligo-glycosid (Fa. Henkel Corp.)

**EP 0 845 978 B1**

Plantaren® 2000 : $C_8$ - $C_{16}$ - Alkyl-oligo-glycosid (Fa. Henkel Corp.)

**Beispielrezepturen für transparente Mikroemulsionskonzentrate**

Tab. 1:

| Beispielrezepturen für transparente Mikroemulsionskonzentrate (in Gew.-%) | | |
|---|---|---|
| | Beispiel 1 | Beispiel 2 |
| Plantaren® 1200 | 11,21 | 0 |
| Plantaren® 2000 | 7,48 | 26,00 |
| Glycerinmonooleat | 4,67 | 0 |
| Dioctylether | 26,17 | 20,00 |
| Octyldodecanol | 6,54 | 0 |
| Triethylcitrat | 0 | 10,00 |
| Parfümöl | 6,54 | 5,0 |
| Wasser | 37,38 | 39,00 |
| Transmission (650 nm, %) | 99 | 95 |

**Beispielrezepturen für sprühfähige, translucente Antitranspirant-Mikroemulsionen**

Tab. 2:

| Beispielrezepturen für sprühfähige, translucente Antitranspirant-Mikroemulsionen (Endzusammensetzung in Gew.-%) | | | |
|---|---|---|---|
| | Beispiel 3 | Beispiel 4 | Beispiel 5 |
| Plantaren® 1200 | 1,71 | 1,71 | 0 |
| Plantaren® 2000 | 1,14 | 1,39 | 2,40 |
| Glycerinmonooleat | 0,71 | 0,71 | 0 |
| Dioctylether | 4,00 | 4,00 | 0,09 |
| Octyldodecanol | 1,00 | 1,00 | 0,02 |
| Parfümöl | 1,00 | 1,00 | 1,00 |
| Aluminiumchlorohydrat | 8,00 | 5,00 | 5,00 |
| 1,2-Propylenglycol | 5,00 | 5,00 | 0 |
| Glycerin | 0 | 0 | 5,00 |
| Wasser | 77,44 | 80,19 | 86,49 |
| Transmission (650 nm, %) | 70,30 | 74,10 | 55,00 |

**Patentansprüche**

1. Translucente Antitranspirantien in Form einer sprühfähigen, feinteiligen Öl-in-Wasser-Emulsion, enthaltend

   a) 0,5 bis 30 Gew.-% einer nicht mit Wasser mischbaren Ölphase,
   b) 40 bis 90 Gew.-% Wasser und
   c) bezogen auf einen Gewichtsteil Ölphase, 0,35 bis 30 Gew.-Teile nichtionischer Emulgatoren, insbesondere

6

aus der Gruppe der Alkylpolyglykoside,
d) gegebenenfalls 0,1 bis 7,5 Gew.-Teile, bezogen auf einen Gewichtsteil Ölphase, eines lipophilen Coemulgators sowie weitere übliche Zusatzstoffe,

**dadurch gekennzeichnet, daß** weiterhin 0,1 bis 25 Gew.-% eines anorganischen, adstringierenden Antitranspirant-Wirkstoffes enthalten sind und die Tröpfchengröße der emulgierten Phase im wesentlichen zwischen 1 und 100 nm liegt.

2. Antitranspirantien nach Anspruch 1, **dadurch gekennzeichnet, daß** die Emulsion 2 bis 15 Gew.-%, insbesondere 2 bis 10 Gew.-% einer nicht mit Wasser mischbaren Ölphase enhält.

3. Antitranspirantien nach einem der Ansprüche 1 oder 2, **dadurch gekennzeichnet, daß** die Emulsion 60 bis 90 Gew.-%, insbesondere 70 bis 88 Gew.-% Wasser enthält.

4. Antitranspirantien nach einem der Ansprüche 1 bis 3, **dadurch gekennzeichnet, daß** die Emulsion 0,5 bis 10 Gew.-Teile, insbesondere 0,6 bis 4 Gew.-Teile, bezogen auf einen Gewichtsteil Ölphase, nichtionischer Emulgatoren, bevorzugt aus der Gruppe der Alkylpolyglycoside enthält.

5. Antitranspirantien nach einem der Ansprüche 1 bis 4, **dadurch gekennzeichnet, daß** ein oder mehrere nichtionische Emulgatoren aus der Gruppe der Alkylpolyglycoside mit einer Kettenlänge des lipophilen Rests zwischen 8 und 16 C-Atomen enthalten sind.

6. Antitranspirantien nach einem der Ansprüche 1 bis 5, **dadurch gekennzeichnet, daß** die Ölphase mindestens 30 Gew.-%, insbesondere mindestens 50 Gew.-% Dialkylether enthält.

7. Antitranspirantien nach einem der Ansprüche 1 bis 6, **dadurch gekennzeichnet, daß** als Coemulgatoren Fettsäure-Polyol-Partialester, Fettalkohole oder Fettalkohol-Polyol-Ether enthalten sind.

8. Antitranspirantien nach einem der Ansprüche 1 bis 7, **dadurch gekennzeichnet, daß** das Verhältnis von nichtionischem Emulgator zu Coemulgator zwischen 1:1 und 20:1, insbesondere zwischen 2:1 und 15:1 liegt.

9. Translucente Mikroemulsionskonzentrate, enthaltend

a) 20 bis 40 Gew.-% Wasser sowie
b) 20 bis 40 Gew.-% eines nicht wasserlöslichen Ölkörpers und
c) bezogen auf einen Gewichtsteil der Ölphase, 0,35 bis 30 Gew.-Teile nichtionischer Emulgatoren, insbesondere aus der Gruppe der Alkylpolyglycoside sowie
d) gegebenenfalls 0,1 bis 10 Gew.-% Parfümöle,

**dadurch gekennzeichnet, daß** die Tröpfchengröße im wesentlichen zwischen 1 und 100 nm liegt.

10. Verfahren zur Herstellung translucenter Mikroemulsionskonzentrate nach Anspruch 9, **dadurch gekennzeichnet, daß** man die wasserlöslichen Komponenten in Wasser, die öllöslichen Komponenten (gegebenenfalls bei erhöhter Temperatur) in der Ölphase löst und die Phasen bei Temperaturen zwischen 10 und 90°C intensiv vermischt.

11. Verfahren zur Herstellung translucenter Antitranspirantien, **dadurch gekennzeichnet, daß** ein translucentes Mikroemulsionskonzentrat gemäß Anspruch 9, mit einer wässrigen Lösung von Antitranspirant-Wirkstoffen intensiv vermischt wird.

**Claims**

1. Translucent antiperspirants in the form of a sprayable fine-droplet oil-in-water emulsion containing

a) 0.5 to 30% by weight of a water-immiscible oil phase,
b) 40 to 90% by weight of water and
c) based on 1 part by weight of oil phase, 0.35 to 30 parts by weight of nonionic emulsifiers, more particularly from the group of alkyl polyglycosides,

d) optionally 0.1 to 7.5 parts by weight - based on 1 part by weight of oil phase - of a lipophilic co-emulsifier and other typical additives,

**characterized in that** the emulsion additionally contains 0.1 to 25% by weight of an inorganic astringent antiperspiration agent and **in that** the droplet size of the emulsified phase is essentially between 10 and 100 nm.

2. Antiperspirants as claimed in claim 1, **characterized in that** the emulsion contains 2 to 15% by weight and, more particularly, 2 to 10% by weight of a water-immiscible oil phase.

3. Antiperspirants as claimed in claim 1 or 2, **characterized in that** the emulsion contains 60 to 90% by weight and, more particularly, 70 to 88% by weight of water.

4. Antiperspirants as claimed in any of claims 1 to 3, **characterized in that** the emulsion contains 0.5 to 10 parts by weight and, more particularly, 0.6 to 4 parts by weight - based on 1 part by weight of oil phase - of nonionic emulsifiers, preferably from the group of alkyl polyglycosides.

5. Antiperspirants as claimed in any of claims 1 to 4, **characterized in that** they contain one or more nonionic emulsifiers from the group of alkyl polyglycosides with a chain length of the lipophilic group of 8 to 16 carbon atoms.

6. Antiperspirants as claimed in any of claims 1 to 5, **characterized in that** the oil phase contains at least 30% by weight and, more particularly, at least 50% by weight of dialkyl ethers.

7. Antiperspirants as claimed in any of claims 1 to 6, **characterized in that** they contain fatty acid polyol partial esters, fatty alcohols or fatty alcohol polyol ethers as co-emulsifiers.

8. Antiperspirants as claimed in any of claims 1 to 7, **characterized in that** the ratio of nonionic emulsifier to co-emulsifier is between 1:1 and 20:1 and, more particularly, between 2:1 and 15:1.

9. Translucent microemulsion concentrates containing

   a) 20 to 40% by weight of water,
   b) 20 to 40% by weight of a water-insoluble oil and
   c) based on 1 part by weight of oil phase, 0.35 to 30 parts by weight of nonionic emulsifiers, more particularly from the group of alkyl polyglycosides, and
   d) optionally 0.1 to 10% by weight of perfume oils,

   **characterized in that** the droplet size is essentially between 1 and 100 nm.

10. A process for producing the translucent microemulsion concentrates claimed in claim 9, **characterized in that** the water-soluble components are dissolved in water while the oil-soluble components are dissolved in the oil phase (optionally at elevated temperature) and the phases are intensively mixed at temperatures of 10 to 90°C.

11. A process for the production of translucent antiperspirants, **characterized in that** the translucent microemulsion concentrate claimed in claim 9 is intensively mixed with an aqueous solution of antiperspiration agents.

**Revendications**

1. Agents antitranspirants translucides sous forme d'une émulsion huile dans l'eau, apte à la pulvérisation, dispersée finement contenant :

   a) de 0,5 à 30 % en poids d'une phase huileuse non miscible à l'eau
   b) de 40 à 90 % en poids d'eau, et
   c) rapporté à une partie en poids de la phase huileuse, de 0,35 à 30 % en poids d'agents émulsionnants non ioniques, en particulier choisis dans le groupe des alkylpolyglycosides, et
   d) éventuellement de 0,1 à 7,5 parties en poids rapporté à une partie en poids de la phase huileuse, d'un agent co-émulsionnant lipophile ainsi que d'autres additifs usuels,

**caractérisés en ce qu'**
en outre de 0,1 à 25 % en poids d'un principe actif minéral, astringent, d'agent antitranspirant sont contenus, et la taille des gouttelettes de la phase émulsionnée se situe essentiellement entre 1 et 100 nm.

**2.** Agents antitranspirants selon la revendication 1,
**caractérisés en ce que**
l'émulsion contient de 2 à 15 % en poids, en particulier de 2 à 10 % en poids d'une phase huileuse, non miscible à l'eau.

**3.** Agents antitranspirants selon l'une des revendications 1 ou 2,
**caractérisés en ce que**
l'émulsion contient de 60 à 90 % en poids, en particulier de 70 à 88 %, en poids d'eau.

**4.** Agents antitranspirants selon l'une des revendications 1 à 3,
**caractérisés en ce que**
l'émulsion contient de 0,5 à 10 parties en poids, en particulier de 0,6 à 4 parties en poids rapporté à une partie en poids de la phase huileuse, d'agents émulsionnants non ioniques choisis de préférence dans le groupe des alkylpolyglycosides.

**5.** Agents antitranspirants selon l'une des revendications 1 à 4,
**caractérisés en ce qu'**
ils contiennent un ou plusieurs agents émulsionnants non ioniques choisis dans le groupe des alkylpolyglycosides ayant une longueur de chaîne du reste lipophile, comprise entre 8 et 16 atomes de carbone.

**6.** Agents antitranspirants selon l'une des revendications 1 à 5,
**caractérisés en ce que**
la phase huileuse contient au moins 30 % en poids, en particulier au moins 50 % d'éther dialkylique.

**7.** Agents antitranspirans selon l'une des revendications 1 à 6,
**caractérisés en ce qu'**
ils contiennent comme agents co-émulsionnants des esters partiels d'acide gras et de polyol, des alcools gras ou des éthers d'alcool gras et de polyol.

**8.** Agents antitranspirants selon l'une des revendications là 7,
**caractérisés en ce que**
le rapport de l'agent émulsionnant non ionique à l'agent co-émulsionnant se situe entre 1 :1 et 20 :1, en particulier entre 2 :1 et 15 : 1.

**9.** Concentrés de microémulsion translucides contenant

    a) de 20 à 40 % en poids d'eau ainsi que
    b) de 20 à 40 % en poids d'un composé huileux qui n'est pas soluble dans l'eau et
    c) rapporté à une partie en poids de la phase huileuse, de 0,35 à 30 parties en poids d'agents émulsionnants non ioniques, en particulier choisis dans le groupe des alkylpolyglycosides ainsi que
    d) éventuellement de 0,1 à 10 % en poids d'essences de parfum,

**caractérisés en ce que**
la taille des gouttelettes se situe essentiellement entre 1 et 100 mn.

**10.** Procédé de production de concentrés de micro-émulsions translucides selon la revendication 9,
**caractérisé en ce qu'**
on dissout dans l'eau les composants solubles dans l'eau, on dissout les composants solubles dans l'huile (le cas échéant à température augmentée) dans la phase huileuse, et on mélange d'une manière intensive les phases à des températures comprises entre 10 et 90°C.

**11.** Procédé de production d'agents antitranspirant translucides,
**caractérisé en ce qu'**
on mélange d'une manière intensive un concentré de micro-émulsion translucide conformément à la revendication 9 avec une solution aqueuse de principes actifs antitranspirants.